(19) Europäisches Patentamt
European Patent Office
Office européen des brevets

(11) **EP 3 598 987 B1**

(12) **EUROPEAN PATENT SPECIFICATION**

(45) Date of publication and mention
of the grant of the patent:
**07.04.2021 Bulletin 2021/14**

(51) Int Cl.:
***A61M 1/16*** *(2006.01)*

(21) Application number: **19187736.4**

(22) Date of filing: **23.07.2019**

(54) **PATIENT PRE-BUN ESTIMATE BASED ON SORBENT RECHARGER EFFLUENT DATA**

SCHÄTZUNG VON PRÄ-BUN EINES PATIENTEN AUF BASIS VON SORPTIONSMITTELAUFLADERAUSSTRÖMUNGSDATEN

ESTIMATION PRÉ-URÉE DE PATIENT BASÉE SUR DES DONNÉES D'EFFLUENT DE RECHARGEUR DE SORBANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.07.2018 US 201862702497 P**
**01.04.2019 US 201916371155**

(43) Date of publication of application:
**29.01.2020 Bulletin 2020/05**

(73) Proprietor: **Medtronic, Inc.**
**Minneapolis, MN 55432 (US)**

(72) Inventors:
• **PUDIL, Bryant J.**
**Plymouth, MN Minnesota 55447 (US)**
• **HOBOT, Christopher M.**
**Rogers, MN Minnesota 55374 (US)**
• **GERBER, Martin T**
**Maple Grove, MN Minnesota 55369 (US)**

(74) Representative: **Maschio, Antonio**
**Maschio & Soames IP Limited**
**30 Carlton Crescent**
**Southampton SO15 2EW (GB)**

(56) References cited:
**EP-A1- 2 950 836       US-A1- 2016 243 541**

**Description**

CROSS-REFERENCE TO RELATED APPLICATION

This application claims the benefit of and priority to U.S. Provisional Patent Application No. 62/702,497 filed July 24, 2018,

FIELD OF THE INVENTION

The invention relates to devices, systems, and methods for estimating a patient blood urea nitrogen (BUN) level prior to a dialysis session based on data received when introducing an ammonium removal solution through a zirconium phosphate sorbent module. The systems and methods can introduce an ammonium removal solution and determine an ammonia content of the ammonium removal solution effluent to estimate the patient pre-dialysis BUN level.

BACKGROUND

Urea is a marker for dialysis adequacy in hemodialysis treatment. To measure a patient's blood urea nitrogen (BUN) level, existing systems and methods usually require a technician to obtain a blood sample from the patient. The technician then uses a blood gas analyzer to analyze the blood sample to determine BUN. The known process for obtaining BUNS is expensive and time-consuming and relies on many manual steps that are not easily automatable. The known systems and methods fail to appreciate that BUN can be estimated by measuring an amount of ammonia absorbed onto zirconium phosphate. In sorbent dialysis, zirconium phosphate is used to remove waste and unwanted solutes including ammonium, potassium, calcium, and magnesium ions from dialysate. Zirconium oxide is used to remove phosphate ions from dialysate. Urease is used to breakdown urea into carbon dioxide and ammonium in order to facilitate their removal. During treatment, the ammonium ions are adsorbed by the zirconium phosphate to avoid being returned to the patient. Known systems and methods, however, cannot determine an amount of ammonium ions absorbed by the zirconium phosphate. The known systems and methods do not provide for such exchange and fail to provide for the measurement of such exchange of ammonium ions for sodium or hydrogen ions. As such, known systems and methods cannot measure the amount of ammonium ions displaced from zirconium phosphate during an exchange process. The systems also cannot take measurements using automated means and must rely on manually drawing blood from a patient to obtain BUN.

Hence, there is a need for systems and methods for determining an amount of ammonia without requiring a patient's blood sample. The need extends to measuring pre-dialysis BUN i.e., pre-BUN levels, using measurements from fluids excluding a patient's blood. The need extends to automated systems and methods for estimating BUN or pre-BUN. There is a further need for measuring an amount of ammonia displaced from zirconium phosphate during a process wherein one or more solutions are passed through the zirconium phosphate. The need extends to systems and methods suitable for use in dialysis and in recharging a sorbent cartridge for use in dialysis. The need includes systems and methods for estimating a patient's pre-dialysis BUN level based on an amount of total ammonia displaced during a recharging process of a sorbent cartridge.

SUMMARY OF THE INVENTION

The first aspect of the invention relates to a method. In any embodiment, the method can comprise introducing one or more ammonium removal solutions into a zirconium phosphate sorbent module; determining a total ammonia content in an ammonium removal solution effluent of the zirconium phosphate sorbent module using an ammonia sensor; and estimating a patient pre-dialysis BUN level based on the total ammonia content in the ammonium removal solution effluent.

In any embodiment, the ammonia sensor can be in an effluent line fluidly connectable to an outlet of the zirconium phosphate sorbent module.

In any embodiment, the method can comprise the step of introducing the ammonium removal solution effluent in a reservoir, and the step of determining the total ammonia content in the ammonium removal solution effluent can comprise determining the total ammonia content in the reservoir.

In any embodiment, the step of estimating the patient pre-dialysis BUN level can comprise determining a total ammonia content of the ammonium removal solution effluent to determine a volume averaged total ammonia content of the ammonium removal solution effluent.

In any embodiment, the step of determining the total ammonia content of the ammonium removal solution effluent can comprise integrating an ammonia content of the ammonium removal solution effluent.

In any embodiment, the method can comprise determining an amount of ammonia removed by the zirconium phosphate sorbent module during a dialysis session.

In any embodiment, the step of estimating the patient pre-dialysis BUN level can using an equation:

$$C_{\text{Burea, pre}} = \frac{(\bar{C}_{NH4} * V_{eff}) / 2}{V_{pre} - V_{post}(1 - URR)},$$ wherein $C_{\text{Burea, pre}}$ is the patient pre-dialysis urea level, $\overline{C}_{NH4}$ is a volume averaged total ammonia content in the ammonium removal solution effluent; $V_{eff}$ is a volume of ammonium removal solutions introduced through the zirconium phosphate sorbent module, $V_{pre}$ is a patient water volume prior to a dialysis session, $V_{post}$ is a patient water volume after the dialysis session, and URR is a urea reduction ratio for the dialysis session.

In any embodiment, the method can use a sorbent recharger and the one or more ammonium removal solutions can comprise one or more recharge solutions; and the step of introducing the one or more recharge solutions through the zirconium phosphate sorbent module can comprise first introducing water through the zirconium phosphate sorbent module and then introducing a brine solution through the zirconium phosphate sorbent module.

In any embodiment, the step of determining the total ammonia content in the ammonium removal solution effluent can comprise determining the total ammonia content while introducing the water through the zirconium phosphate sorbent module and determining the total ammonia content while introducing the brine solution through the zirconium phosphate sorbent module.

In any embodiment, the step of determining the total ammonia content in the ammonium removal solution effluent can comprise determining the total ammonia content while introducing the brine solution through the zirconium phosphate sorbent module.

In any embodiment, the step of determining the total ammonia content in the ammonium removal solution effluent can comprise continuously determining the total ammonia content in the ammonium removal solution effluent.

In any embodiment, the step of determining the total ammonia content in the ammonium removal solution effluent can comprise determining the total ammonia content in the ammonium removal solution effluent at preset intervals.

In any embodiment, a processor can be programmed to estimate the patient pre-dialysis BUN level based on the ammonia content in the ammonium removal solution effluent.

The features disclosed as being part of the first aspect of the invention can be in the first aspect of the invention, either alone or in combination, or follow a preferred arrangement of one or more of the described elements.

The second aspect of the invention is drawn to a system. In any embodiment, the system can comprise a flow path comprising: at least one ammonium removal solution source; the ammonium removal solution source fluidly connectable to a zirconium phosphate module inlet; a pump; and an effluent line fluidly connectable to a zirconium phosphate module outlet; at least one ammonia sensor; and a processor in communication with the at least one ammonia sensor, the processor estimating a patient pre-dialysis BUN level based on a total ammonia content in an ammonium removal solution effluent of the zirconium phosphate sorbent module.

In any embodiment, the system can comprise a sorbent recharger; and the at least one ammonium removal solution source can comprise at least a water source and a brine source.

In any embodiment, the processor can determine an amount of ammonia removed by the zirconium phosphate sorbent module.

In any embodiment, the processor can estimate the patient pre-dialysis BUN level using an equation:

$$C_{\text{Burea, pre}} = \frac{(\bar{C}_{NH4} * V_{eff}) / 2}{V_{pre} - V_{post}(1 - URR)},$$ wherein $C_{\text{Burea, pre}}$ is the patient pre-dialysis urea level, $\overline{C}_{NH4}$ is a volume averaged total ammonia content in the ammonium removal solution effluent; $V_{eff}$ is a volume of ammonium removal solutions introduced through the zirconium phosphate sorbent module, $V_{pre}$ is a patient water volume prior to a dialysis session, $V_{post}$ is a patient water volume after the dialysis session, and URR is a urea reduction ratio for the dialysis session.

In any embodiment, the processor can be programmed to determine the urea reduction ratio using an equation: URR = 1 - $e^{-kt/v}$, wherein k is a dialyzer clearance, t is a length of time of a dialysis session, and v is a patient water volume.

In any embodiment, the processor can be programmed to receive the total ammonia content of the ammonium removal solution effluent from the at least one ammonia sensor continuously during the method.

In any embodiment, the processor can be programmed to receive the total ammonia content of the ammonium removal solution effluent from the at least one ammonia sensor at preset intervals.

In any embodiment, the processor can control the pump to first introduce water from the water source through the zirconium phosphate sorbent module, and then introduce brine from the brine source through the zirconium phosphate sorbent module.

In any embodiment, the processor can be programmed to receive the total ammonia content of the ammonium removal solution effluent while water is introduced through the zirconium phosphate sorbent module and while brine is introduced through the zirconium phosphate sorbent module.

In any embodiment, the processor can be programmed to receive the total ammonia content of the ammonium removal solution effluent while brine is introduced through the zirconium phosphate sorbent module.

In any embodiment, the system can comprise a reservoir fluidly connectable to the effluent line, the at least one ammonia sensor determining the total ammonia content of the ammonium removal solution effluent in the reservoir.

In any embodiment, the at least one ammonia sensor can be in the effluent line.

In any embodiment, the system can comprise a temperature sensor in communication with the processor.

The features disclosed as being part of the second aspect of the invention can be in the second aspect of the invention, either alone or in combination, or follow a preferred arrangement of one or more of the described elements.

## BRIEF DESCRIPTION OF THE DRAWINGS

FIG. 1 is a flow path for removing ammonia from a sorbent module.

FIG. 2 is a flow chart illustrating a method of estimating a patient pre-dialysis BUN level based on ammonia removed from a sorbent cartridge.

FIG. 3 is a flow path for recharging zirconium phosphate in a sorbent module.

FIG. 4 is a flow chart illustrating a method for estimating a patient pre-dialysis blood urea nitrogen level based on data from an effluent recharger solution.

## DETAILED DESCRIPTION OF THE INVENTION

Unless defined otherwise, all technical and scientific terms used have the same meaning as commonly understood by one of ordinary skill in the art.

The articles "a" and "an" are used to refer to one or to over one (*i.e.*, to at least one) of the grammatical object of the article. For example, "an element" means one element or over one element.

A "ammonia sensor" can be any component or set of components capable of determining a concentration of ammonia within a fluid. In certain embodiments an ammonia sensor can determine both an ammonia and an ammonium ion concentration in a fluid.

An "ammonium removal flow path" can be a path through which fluid can travel while removing ammonium ions from a material. One non-limiting material can be a sorbent material.

An "ammonium removal solution" is any solution containing solutes that promote the release of ammonium ions from a material. One non-limiting material can be a sorbent material.

The term "ammonium removal solution source" refers to a source of a solution that contains solutes that can promote release of ammonium ions from a material, such as a sorbent material. In certain embodiments, the ammonium removal solution source can be an acid, base, sodium ions, potassium ions, calcium ions, magnesium ions, or combinations thereof.

The term "amount of ammonia removed" can refer to a total amount of ammonia or ammonium ions taken out of a fluid or solution.

A "brine solution" can be a solution containing salts and/or buffers containing solutes used in recharging a material such as a sorbent material. In certain embodiments, the brine solution can be a solution of a sodium salt, acetic acid, sodium acetate, or combinations thereof.

The term "brine source" refers to a source of a solution of salts and/or buffers containing solutes used in recharging a sorbent material. In certain embodiments, the brine source can contain a sodium salt, acetic acid, sodium acetate, or combinations thereof.

The terms "communication" or "electronic communication" can refer to the ability to transmit electronic data, instructions, information wirelessly, via electrical connection, or any other electrical transmission between two components or systems.

The term "comprising" includes, but is not limited to, whatever follows the word "comprising." Use of the term indicates that the listed elements are required or mandatory but that other elements are optional and may be present.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The term "continuously," when referring to a frequency of measurements, can refer to taking measurements without halting during a process.

The terms "control," "controlling," or "controls" refer to the ability of one component to direct the actions of a second or more than one other component.

The terms "determining" and "determine" can refer to ascertaining or identifying a particular state or desired state. For example, a system or fluid, or any measured variable(s) or feature(s) of a system or a fluid can be determined by obtaining sensor data, retrieving data, performing a calculation, or by any other known method.

A "dialysis session" can be any time period of any length during which a patient is treated by or undergoes dialysis, hemodialysis, hemofiltration, ultrafiltration, or other fluid removal therapy.

The term "dialyzer clearance" refers to a rate at which solutes pass through a dialyzer membrane.

The term "effluent" can refer to liquid, gas, or a combination thereof exiting a container, compartment, or cartridge.

An "effluent line" can be a fluid passageway, tube, or path of any kind into which liquid, gas, or a combination thereof exiting a container, module, or component can flow.

"Estimated," "estimating," to "estimate," or "estimation" can each refer to a determination of one or more parameters indirectly using one or more variables.

The term "fluidly connectable" refers to a capability for providing for the passage of fluid, gas, or combination thereof, from one point to another point. The capability for providing such passage can be any connection, fastening, or forming between two points to permit the flow of fluid, gas, or combinations thereof. The two points can be within or between any one or more of compartments of any type, modules, systems, components, such as rechargers, as described herein.

The term "fluidly connected" refers to a particular state such that the passage of fluid, gas, or combination thereof, is provided from one point to another point. The connection state can also include an unconnected state, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

The term "integrating" or to "integrate" refers to determining a total area under a curve for a particular function.

The terms "introducing," "introduced," or to "introduce" refers to directionally moving or flowing a fluid, a gas, or a combination thereof by any means known to those of skill in the art.

The term "length of time of a dialysis session" refers to the total amount of time from the beginning to end of a single dialysis session.

A "patient" or "subject" can be a member of any animal species, preferably a mammalian species, optionally a human. The subject can be an apparently healthy individual, an individual suffering from a disease, or an individual being treated for a disease. In certain embodiments, the patient can be a human, sheep, goat, dog, cat, mouse or any other animal.

The term "patient pre-dialysis blood urea nitrogen (BUN) level" can refer to the amount of nitrogen that comes from urea that is within the body of a patient prior to a dialysis session. The BUN measurement is generally given in units of mg/dl, but can also be given in units of millimoles per liter urea, or any other units of concentration.

The term "patient pre-dialysis urea level" can refer to the amount of urea within the body of a patient prior to a dialysis session. The BUN measurement is generally given in units of millimoles per liter, but can also be given in any other units of concentration.

"Patient water volume" refers to the total amount of water in a patient.

A "preset interval" or "present intervals" can be a period of time between events that is determined prior to the events.

The term "processor" as used is a broad term and is to be given its ordinary and customary meaning to a person of ordinary skill in the art. The term refers without limitation to a computer system, state machine, processor, or the like designed to perform arithmetic or logic operations using logic circuitry that responds to and processes the basic instructions that drive a computer. In any embodiment of the first, second, third, and fourth invention, the terms can include ROM ("read-only memory") and/or RAM ("random-access memory") associated therewith.

The term "programmed," when referring to a processor, can mean a series of instructions that cause a processor to perform certain steps.

The term "pump" refers to any device that causes the movement of fluids or gases by applying suction or pressure.

The term "receiving" refers to obtaining information or data from any source.

A "recharge solution" or "recharge solutions" can be a solution containing appropriate ions for recharging a specific sorbent material. A recharge solution can be a single solution containing all necessary ions for recharging a sorbent material. Alternatively, the recharge solution can contain some of the ions for recharging the sorbent material, and one or more other recharge solutions can be used to form a composite "recharge solution" to recharge the sorbent material, as described herein.

A "recharge solution source" can be any fluid or concentrate source from which a recharge solution can be stored, obtained, or delivered therefrom.

"Recharging" refers to treating a sorbent material to restore a functional capacity of the sorbent material putting the sorbent material back into a condition for reuse or use in a new dialysis session. In some instances, the total mass, weight and/or amount of "rechargeable" sorbent materials remain the same. In some instances, the total mass, weight and/or amount of "rechargeable" sorbent materials change. Without being limited to any one theory of invention, the recharging process may involve exchanging ions bound to the sorbent material with different ions, which in some instances may increase or decrease the total mass of the system. However, the total amount of the sorbent material will in some instances be unchanged by the recharging process. Upon a sorbent material undergoing "recharging," the sorbent material can then be said to be "recharged."

A "reservoir" is a container or component that can hold a liquid, fluid, gas, or combination thereof.

A "sorbent cartridge module" or "sorbent module" means a discreet component of a sorbent cartridge. Multiple sorbent cartridge modules can be fitted together to form a sorbent cartridge of two, three, or more sorbent cartridge modules. The "sorbent cartridge module" or "sorbent module" can contain any selected materials for use in sorbent dialysis and may or may not contain a "sorbent material" or adsorbent, but less than the full complement of sorbent materials needed. In other words, the "sorbent cartridge module" or "sorbent module" generally refers to the use of the "sorbent cartridge module" or "sorbent module" in sorbent-based dialysis, e.g., REDY (REcirculating DYalysis), and not that a "sorbent material" that is necessarily contained in the "sorbent cartridge module" or "sorbent module."

A "sorbent recharger" or "recharger" is an apparatus designed to recharge at least one sorbent material.

The term "temperature sensor" refers to a device for measuring the temperature of a gas, a liquid, or a combination thereof in a vessel, container, or fluid line.

The term "total ammonia content" can refer to an amount of ammonia dissolved in a fluid. The total ammonia content can refer to the concentration, mass, or any other value of the amount of ammonia dissolved in the fluid. In certain embodiments, the term ammonia content can refer to the sum of the ammonia content and/or ammonium ion content of a fluid.

"Urea reduction ratio" or "URR" refers to the amount by which the urea level of a patient is reduced during treatment. The URR can be expressed as 1 minus the ratio of the patient's ending urea level over the patient's starting urea level.

The term "volume averaged total ammonia content" can refer to a total ammonia content of a fluid divided by a total volume of the fluid.

A "water source" can be a fluid source from which water can be stored, obtained, or delivered therefrom.

"Zirconium phosphate" is a sorbent material that removes cations from a fluid, exchanging the removed cations for different cations.

The term "zirconium phosphate module inlet" can refer to a portion of a sorbent module containing zirconium phosphate through which fluid, gas, or a combination thereof can be drawn into the sorbent module.

The term "zirconium phosphate module outlet" can refer to a portion of a sorbent module containing zirconium phosphate through which fluid, gas, or a combination thereof can be drawn out of the sorbent module.

*Zirconium Phosphate Ammonium Removal*

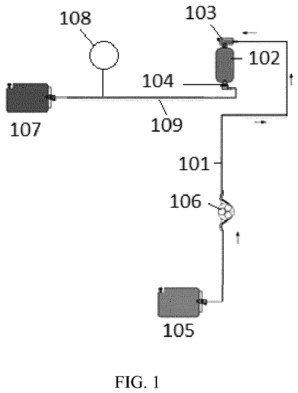

The invention is drawn to systems and methods for estimating a patient pre-dialysis blood urea nitrogen (BUN) level based on data received during removal of ammonium ions from a sorbent module containing zirconium phosphate used by the patient during a previous dialysis session. FIG. 1 illustrates a non-limiting embodiment of the system. The system can include an ammonium removal flow path **101** fluidly connectable to an ammonium removal solution source **105**. The ammonium removal solution source **105** can contain a solution of one or more solutes that will displace ammonium ions bound to zirconium phosphate in a zirconium phosphate sorbent module **102** during treatment. During a dialysis session, the zirconium phosphate can remove cations from spent dialysate, including ammonium, potassium, calcium, and magnesium, exchanging the cations for hydrogen and sodium ions. The ammonia is formed by the breakdown of urea by urease in the sorbent cartridge during treatment. The amount of ammonia adsorbed by the zirconium phosphate is therefore a function of the amount of urea removed during treatment. The ammonium removal solution in ammonium removal solution source **105** can contain any solutes that will displace the ammonium ions from the zirconium phosphate. In certain embodiments, the ammonium removal solution can contain sodium ions, calcium ions, magnesium ions, potassium ions, acid, or base. Because calcium and magnesium bind more strongly to the zirconium phosphate, an ammonium removal solution containing calcium or magnesium may be used when the zirconium phosphate sorbent module **102** is single use.

The zirconium phosphate sorbent module **102** can be fluidly connectable to the ammonium removal flow path **101** through zirconium phosphate module inlet **103** and zirconium phosphate module outlet **104.** In certain embodiments, the zirconium phosphate sorbent module **102** can be reusable. Alternatively, the zirconium phosphate sorbent module **102** can be single-use. Pump **106** provides a driving force for moving fluids through the flow path. Ammonium ions displaced when solutes from the ammonium removal solution are adsorbed by the zirconium phosphate exit the zirconium phosphate sorbent module **102** through zirconium phosphate module outlet **104** into effluent line **109**. In certain embodiments, an ammonia sensor **108** can measure the total ammonia concentration of the ammonium removal solution effluent in effluent line **109.** As described, a processor in communication with the ammonia sensor **108** can use the total ammonia content of the ammonium removal solution effluent to estimate the patient pre-dialysis BUN level. Alternatively, or additionally, the ammonium removal solution effluent can be collected in an effluent reservoir **107.** The ammonium removal solution effluent collected in effluent reservoir **107** can be pooled and tested to measure the total ammonia content of the ammonium removal solution effluent.

Although shown as a single ammonia sensor **108** in FIG. 1, the ammonia sensor **108** can alternatively be multiple sensors that determine individual parameters of the ammonium removal solution effluent to allow for calculation of the total ammonia content in the ammonium removal solution effluent. For example, the ammonia sensor **108** can be a combination of pH and ammonia sensors, a combination of pH and ammonium ion sensors, a combination of ammonia and ammonium ion sensors, or any one or more sensors that allow for calculation of the total ammonia content of the ammonium removal solution effluent. In certain embodiments, conductivity sensors can be used to measure the total ammonia content of the ammonium removal solution effluent. The ammonia sensor **108** can detect the total ammonia concentration in the ammonium removal solution effluent by any means. For example, one or more ammonia sensing membranes can be contacted with the ammonium removal solution effluent, the ammonia sensing membranes changing color or any other optical parameter in response to the ammonia content in the ammonium removal solution effluent. The optical change in the ammonia sensing membrane can be detected by a photodetector to determine the ammonia content, ammonium content, pH, or combinations thereof. Any number of sensing membranes for determining the ammonia content, ammonium content, or pH can be included in the ammonia sensor **108.** In certain embodiments, the ammonia sensor **108** can measure the partial pressure of ammonia gas in the ammonium removal solution effluent and then use Henry's law and the Henderson-Hasselbach equation to determine the total ammonia content. Alternatively, the ammonia sensor **108** can be an ion selective electrode measuring the ammonium ions in the ammonium removal solution effluent. Additional sensors, such as a temperature sensor (not shown) can be included to determine the total ammonia content of the ammonium removal solution effluent with methods that require the temperature to be known.

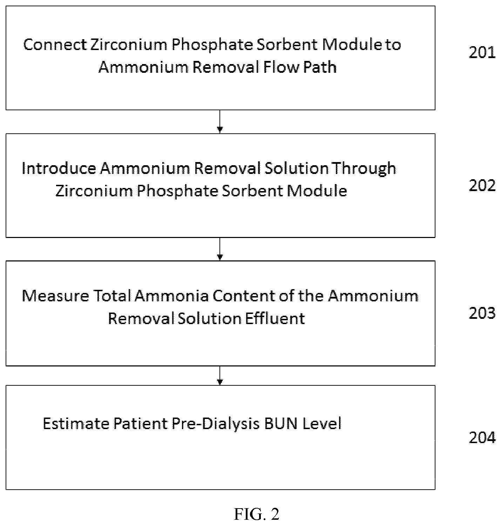

FIG. 2 is a flow chart illustrating the method of estimating the patient pre-dialysis BUN level. In step **201,** a zirconium phosphate sorbent module that was used in a previous dialysis session can be placed into a system for removing ammonium ions by fluidly connecting an inlet and outlet of the zirconium phosphate sorbent module to a flow path as illustrated in FIG. 1. In step **202,** an ammonium removal solution can be introduced through the zirconium phosphate sorbent module. As described, the ammonium removal solution can contain an acid, a base, sodium ions, potassium ions, calcium ions, magnesium ions, or any other ions that can displace the ammonium ions bound to the zirconium phosphate. In step **203,** the processor can receive the total ammonia content in the ammonium removal solution effluent from an ammonia sensor either continuously or at preset intervals. In certain embodiments, the processor can receive the total ammonia content in the ammonium removal solution effluent at preset intervals of between 1 second and 5 minutes, including between 1 second and 30 seconds, between 1 second and 1 minute, between 30 seconds and 2 minutes, between 1 minute and 3 minutes, or between 2 minutes and 5 minutes. In certain embodiments, the processor can receive the total ammonia content of the ammonium removal solution effluent a single time and compare the total ammonium content of the ammonium removal solution effluent with known or characterized discharge/capacity curves using a lookup table or other method of comparison. The flow rate of the ammonium removal solution introduced through the zirconium phosphate sorbent module can also be received by the processor. Using the flow rates and total ammonia contents in the ammonium removal solution effluent, the processor can determine the volume averaged total ammonia content of the effluent. The processor can then estimate the patient pre-dialysis BUN level using the EQ's(1-9) provided herein, as described based on data received from the ammonia sensor in step **204**. Alternatively, the ammonium removal solution effluent can be collected in a reservoir, and the total ammonia content measured in the reservoir. The total ammonium ions removed can be determined by multiplying the total ammonia content of the collected ammonium removal solution effluent by the total volume of ammonium removal solution used.

*Zirconium Phosphate Recharging*

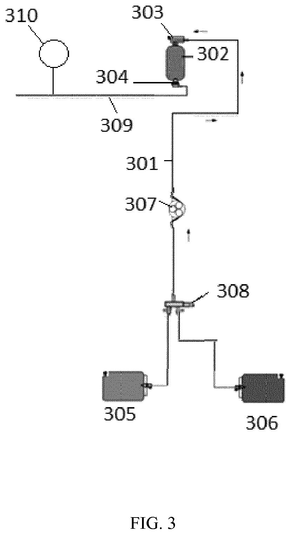

The amount of ammonium ions adsorbed by the zirconium phosphate can also be determined while recharging the zirconium phosphate in a zirconium phosphate sorbent module. The recharging of the sorbent module containing zirconium phosphate can be performed as in U.S. Patent application 14/642,847 (US20150367055A1), the contents of which are incorporated herein in their entirety. FIG. 3 illustrates a non-limiting embodiment of a zirconium phosphate recharging flow path **301** for recharging zirconium phosphate in a zirconium phosphate sorbent module **302.** The recharging flow path **301** illustrated in FIG. 3 can also serve as an ammonium removal flow path for removing ammonium ions from the zirconium phosphate sorbent module **302.** In certain embodiments, the zirconium phosphate sorbent module **302** can be reusable. The zirconium phosphate sorbent module **302** can be fluidly connectable to the zirconium phosphate recharging flow path **301** through zirconium phosphate module inlet **303** and zirconium phosphate module outlet **304.** Pump **307** provides a driving force for moving fluids through the zirconium phosphate recharging flow path **301.** The zirconium phosphate recharging flow path **301** can include one or more recharge solution sources, including a brine source **305** and a water source **306.** The brine source **305** can contain a brine solution of a salt, such as sodium chloride, and a buffer, such as a mixture of sodium acetate and acetic acid. The recharge solutions act as the ammonium removal solution, with the sodium and hydrogen ions in the recharge solution displacing the ammonium ions adsorbed by the zirconium phosphate during a prior dialysis session. Although shown as a single brine source **305,** multiple recharge solution sources can be used. For example, a first recharge solution source containing sodium chloride and a second recharge solution source containing an acetate buffer. Alternatively, three recharge solution sources can be used, with sodium chloride, sodium acetate, and acetic acid in separate recharge solution sources. If multiple recharge solution sources are used, the recharge solutions can be mixed within the zirconium phosphate recharging flow path **301,** or introduced through the zirconium phosphate sorbent module **302** sequentially. Any combination of sodium salt and buffer capable of causing exchange of ammonium, potassium, calcium, and magnesium for sodium and hydrogen ions can be used as the recharge solutions. Optional valve **308** can be included to control the movement of fluid from either the brine source **305** or water source **306.** Alternatively, separate pumps on fluid lines fluidly connected to each recharge solution source can be used. A processor (not shown) can be programmed to control the pumps or valves to direct recharge solutions from the recharge solution sources through the zirconium phosphate sorbent module **302.** One of skill in the art will understand that multiple pump and valve arrangements can be used to introduce the necessary recharge solutions through the zirconium phosphate sorbent module **302.**

During a dialysis session, the zirconium phosphate serves to remove cations from spent dialysate, including ammonium, potassium, calcium, and magnesium, exchanging the cations for hydrogen and sodium ions. The sodium chloride and buffer solutions used in recharging the zirconium phosphate serve to displace the cations absorbed during treatment with sodium and hydrogen ions, facilitating reuse of the zirconium phosphate.

The ammonia is formed by the breakdown of urea by urease in the sorbent cartridge during treatment. The amount of ammonia adsorbed by the zirconium phosphate is therefore a function of the amount of urea removed during treatment. Displaced ammonia from the zirconium phosphate sorbent module **302** will exit the zirconium phosphate sorbent module **302** through zirconium phosphate module outlet **304** into effluent line **309.** An ammonia sensor **310** can determine the total ammonia content of the effluent recharge solution, which is similar to the ammonium removal solution effluent described with respect to FIG.'s 1-2, in effluent line **309.** During the ammonium removal process, the ammonium removal solution can be introduced through the zirconium phosphate sorbent module **302** in either direction. The ammonium removal solution can be introduced through the zirconium phosphate sorbent module **302** in the same direction as the dialysate flow during therapy, or in the reverse direction. Generally, when recharging the zirconium phosphate sorbent module **302,** the direction of flow of the recharge solutions is in the reverse direction as compared to the dialysate flow during treatment. However, when the ammonium removal solution is introduced in the same direction as the dialysate flow during therapy, the amount of time required before ammonia is detected in the effluent can provide an indication of the amount of ammonium ions removed by the zirconium phosphate sorbent module **302** during therapy. Generally, only the most proximal portion of the zirconium phosphate sorbent module **302** will be saturated with ammonium ions during therapy and the distance ammonium ions travel through the zirconium phosphate sorbent module **302** during therapy is a function of the amount of ammonium ions removed. The length of time during ammonium removal prior to detection of ammonia in the effluent is a function of the amount of ammonium ions removed during therapy.

One of skill in the art will understand that several methods can be used to determine the total ammonia content in the effluent line **309.** In certain embodiments, the ammonia sensor **310** can determine concentrations of both ammonia and ammonium ions in the effluent line **309.** Alternatively, the ammonia sensor **310** can determine either the ammonia or ammonium ion concentration and the pH, allowing the total ammonia content to be determined using the Henderson-Hasselbach equation. In certain embodiments, the ammonia sensor **310** can measure the partial pressure of ammonia gas, with the total ammonia content of the effluent determined using Henry's law and the Henderson-Hasselbach equation. Additional sensors, such as a temperature sensor can also be used. Although shown as a single ammonia sensor **310** in FIG. 3, the ammonia sensor **310** can alternatively be multiple sensors that determine individual parameters of the effluent recharge solution to allow for calculation of the total ammonia content in the effluent recharge solution. The ammonia sensor **310** can be in communication with a processor (not shown) programmed to estimate the patient pre-dialysis BUN level based on data received from the ammonia sensor **310.** In certain embodiments, an effluent reservoir (not shown) can be fluidly connectable to effluent line **309.** The total ammonia content of the collected effluent can be measured to determine the total amount of ammonium ions removed from the zirconium phosphate during recharging.

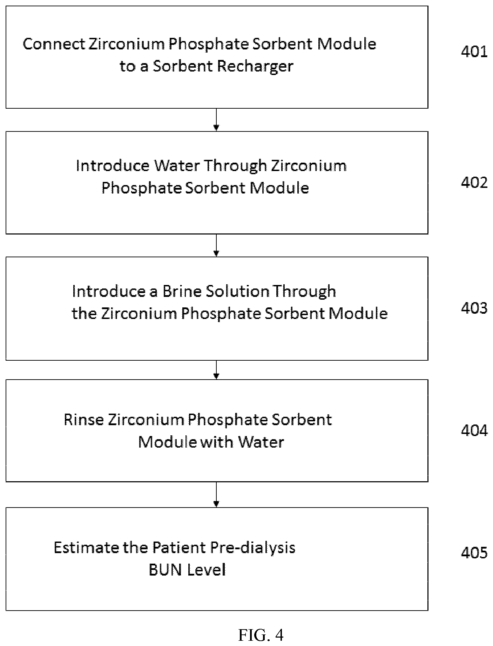

FIG. 4 illustrates a flow chart for the method of estimating the patient pre-dialysis BUN level during recharging. In step **401,** a zirconium phosphate sorbent module that was used in a previous dialysis session can be placed in a receiving compartment of a sorbent recharger and fluidly connected to a zirconium phosphate module inlet and a zirconium phosphate module outlet, as illustrated in FIG. 3. In step **402,** water from a water source can be introduced through the zirconium phosphate sorbent module to rinse the zirconium phosphate sorbent module. In a preferred embodiment, the processor can begin receiving the total ammonia content in the effluent recharge solution during step **402.** However, in certain embodiments, the determining an amount of ammonia can begin after introducing, pumping, or flowing a brine solution through the zirconium phosphate sorbent module in step **403**. As described, the brine solution can contain a sodium salt and a buffer. The brine solution can be introduced or pumped through the zirconium phosphate sorbent module in step **403** as a single solution, or alternatively any combination of sodium salt, acid, and base can be introduced or pumped through the zirconium phosphate sorbent module sequentially. In step **404**, water can be again pumped or introduced through the zirconium phosphate sorbent module to remove the brine solution still remaining in the zirconium phosphate sorbent module. The processor can receive the total ammonia content in the effluent recharge solution from the ammonia sensor either continuously, at preset intervals, or a single time. When a single total ammonia content determination is made the processor can compare the ammonium content of the effluent at the single point in time with known or characterized discharge/capacity curves using a lookup table or other method of comparison. When total ammonia determinations are made at preset intervals the processor can compare the ammonium content of the effluent at the intervals with known curves, providing a higher level of accuracy and confidence in the calculations. With continuous ammonia content determinations, or with determinations in smaller intervals, the processor can integrate the total ammonia content of the effluent to calculate the amount of ammonium ions removed from the zirconium phosphate, or compare the multiple total ammonia measurements with known curves and a high degree of confidence. In certain embodiments, the processor can receive the total ammonia content in the effluent recharge solution at preset intervals of between 1 second and 5 minutes, including between 1 second and 30 seconds, between 1 second and 1 minute, between 30 seconds and 2 minutes, between 1 minute and 3 minutes, or between 2 minutes and 5 minutes. The flow rate of the recharging solutions introduced through the zirconium phosphate sorbent module can also be received by the processor. Using the flow rates and total ammonia contents in the sorbent recharger effluent, the processor can determine the volume averaged total ammonia content. The processor can then estimate the patient pre-dialysis BUN level using the EQ's(1-9), as described based on data received from the ammonia sensor in step **405.**

*Patient BUN Estimation*

Based on data received from the ammonia sensor in the effluent line of the ammonium removal or recharging flow path, or based on the total ammonia content of collected effluent in a reservoir, the processor can integrate the total ammonia content of the ammonium removal solution effluent at each point in time during the process to determine the total amount of ammonia removed from the zirconium phosphate sorbent module, which will equal the total amount of ammonia removed by the zirconium phosphate during the previous dialysis session. The zirconium phosphate sorbent module can be flushed or back flushed with an ammonium removal solution either during recharging of the zirconium phosphate sorbent module, or with a standalone apparatus for introducing an ammonium removal solution through the zirconium phosphate sorbent module. The total ammonia in the ammonium removal solution effluent can be determined as a volume averaged total ammonia content in the effluent line or the total ammonia content of the collected effluent. The total ammonia removed from the zirconium phosphate sorbent module is twice the amount of urea removed by the sorbent cartridge during the dialysis session, as each molecule of urea produces two molecules of ammonia. The total amount of urea removed by the sorbent cartridge can be approximated as equal to the total amount of urea fed through the sorbent cartridge during treatment times the average conversion of urea by the urease within the sorbent cartridge, as shown in EQ(1).

$$\text{Total urea} = (\text{total ammonia}) / 2X \qquad\qquad EQ(1)$$

Where X is equal to the average urea conversion in the sorbent cartridge. The total urea in the dialysate during the dialysis session is given by EQ(2).

$$\text{Total urea} = Q_d * t * \overline{C}_{Durea} \qquad\qquad EQ(2)$$

Where $Q_d$ is the dialysate flow rate, t is the length of the dialysis session, and $\overline{C}_{Durea}$ is the average urea concentration in the dialysate. EQ(3) provides an alternative method for determining the amount of total urea in the dialysate during the dialysis session.

$$\text{Total urea} = V_{pre} * C_{Burea,\,pre} - V_{post} * C_{Burea,\,post} \qquad\qquad EQ(3)$$

Where $V_{pre}$ is a patient water volume prior to the dialysis session (which can be given in units of liters), $V_{post}$ is the patient water volume after the dialysis session (which can be given in units of liters), $C_{Burea,\,pre}$ is the patient urea level prior to the dialysis session (which can be given in units of mmol/L), and $C_{Burea, post}$ is the patient blood urea level after the dialysis session (which can be given in units of mmol/L). One of skill in the art will understand that the pre-dialysis urea level can be easily converted to a pre-dialysis BUN level, and vice versa. Although example units are given for $V_{pre}$, $V_{post}$, and $C_{Burea}$, one of skill in the art will understand that other units can be used. The patient water volume before the dialysis session can be determined via bioimpedance, or estimated based on the patient weight. In certain embodiments, the patient water volume prior to the dialysis session can be assumed as 0.58 * the patient weight. After the dialysis session, the patient water volume can be determined by bioimpedance, estimated based on weight, or determined by the pre-session patient water volume minus the total ultrafiltration during the dialysis session. $C_{Burea, post}$ can be estimated based on the urea reduction ratio (URR), as shown in EQ(4).

$$URR = 1 - \frac{CBurea,post}{CBurea,pre} \text{ or } C_{Burea, post} = URR * C_{Burea, pre} \qquad\qquad EQ(4)$$

The urea reduction ratio can be estimated based on patient volume, dialyzer clearance, and session time, as shown in EQ(5), and is a dimensionless unit.

$$URR = 1 - e^{-kt/V} \qquad\qquad EQ(5)$$

Where k is the dialyzer clearance, t is the length of time of the dialysis session, and v is the patient volume. The dialyzer clearance can be determined using EQ(5).

$$k = \frac{e^S - 1}{\frac{e^S}{Q_B} - \frac{1}{Q_d}} \; ; \; S = \frac{K_o A \left(1 - \frac{Q_B}{Q_D}\right)}{Q_B} \qquad\qquad EQ(6)$$

Where $Q_B$ is the blood flow rate during the dialysis session, $Q_D$ is the dialysate flow rate during the dialysis session, and $K_o A$ is the dialyzer mass transfer coefficient, which can be obtained from the dialyzer specification sheet. Alternatively, the dialyzer clearance can be determined with online clearance monitoring, using techniques known in the art. In certain embodiments, a bolus of NaCl can be added to the dialysate, and the conductivity delta across the dialyzer can be used to estimate clearance. However, any known methods of determining the dialyzer clearance can be used.
As described, the total ammonia removed by the zirconium phosphate can be determined by EQ(7).

$$Total\ NH_4 = \overline{C}_{NH4,\ eff} * V_{eff} \qquad\qquad EQ(7)$$

Where $\overline{C}_{NH4,\ eff}$ is a volume averaged total ammonia content in the ammonium removal solution effluent (which can be given in units of mmol/L), and $V_{eff}$ is the total volume of ammonium removal solution (which can be given in units of liters). Although example units are given for $\overline{C}NH4$ and $V_{eff}$, one of skill in the art will understand that other units can be used. As described, the total $NH_4$ can also be measured by measuring the total ammonia content of effluent collected in a reservoir or container. The total urea removed by the sorbent cartridge is given by EQ(8).

$$Total\ urea = \frac{Total\ NH_4}{2X} \qquad\qquad EQ(8)$$

Plugging the total urea equations into EQ's(1-6) and rearranging allows for estimation of the patient pre-dialysis urea level, $C_{Burea, pre}$, as shown in EQ(9).

$$C_{Burea,\ pre} = \frac{total\ urea}{V_{prp} - V_{post}(1 - URR)} = \frac{Total\ NH_4 / 2X}{V_{prp} - V_{post}(1 - URR)} = \frac{\overline{C}NH4,eff * Veff /2X}{V_{prp} - V_{post}(1 - URR)} \qquad EQ(9)$$

*Example 1*

As a non-limiting example of the patient pre-dialysis BUN estimation using a blood flow rate during a dialysis session as 0.3 L/min and a dialysate flow rate of 0.5 L/min and a dialyzer mass transfer coefficient (KoA) of 1.1-L/min, EQ(6) provides a dialyzer clearance of 0.2679 L/min. Assuming a patient weight of 80 kg, the patient water volume prior to dialysis can be assumed as 0.58 * 80 kg, or 46.4 L. During a 240 minute dialysis session, the URR can be calculated as URR = URR = 1 - e$^{-(0.2679)(240)/46.4}$ = 0.750. An ultrafiltration volume during the dialysis session of 2.0 L would result in a patient post-dialysis water volume of 44.4 L.

Assuming a volume averaged total ammonia content in the effluent of 400 mM, a total effluent volume of 6.0 L, and an average urea conversion by the sorbent cartridge of 0.90, EQ(9) provides the patient pre-dialysis BUN estimation

$$C_{Burea,\, pre} = \frac{400 * 6.0 \,/(2*0.9)}{46.4 - 44.4\,(1 - 0.75)} = 38 \text{ mM}$$

as of urea.

One of skill in the art will understand that the values used in the example patient pre-dialysis BUN estimation are for illustrative purposes only. Actual values for actual patients will vary. However, EQ's(1-9) can be used with any starting values to provide an estimation of the patient pre-dialysis BUN level.

In certain cases, where the ammonium capacity of the zirconium phosphate is exceeded, ammonia breakthrough may occur. The dialysis system can detect ammonia breakthrough with an ammonia sensor in the dialysate flow path and can record the time of ammonia breakthrough. The patient pre-dialysis BUN level can still be estimated using EQ's(1-9) as described with a known dialysate flow rate and time of ammonia breakthrough.

**Claims**

1. A system, comprising:

    an ammonium removal flow path (101) comprising: at least one ammonium removal solution source (105); the ammonium removal solution source fluidly connectable to a zirconium phosphate sorbent module (102) inlet (103); a pump (106); and an effluent line (109) fluidly connectable to a zirconium phosphate module outlet (104); at least one ammonia sensor (108); and
    a processor in communication with the at least one ammonia sensor, the processor estimating a patient pre-dialysis BUN level based on a total ammonia content in an ammonium removal solution effluent of the zirconium phosphate sorbent module.

2. The system of claim 1, wherein the system comprises a sorbent recharger; and
wherein the at least one ammonium removal solution source comprises at least a water source (306) and a brine source (305).

3. The system of claims 1 or 2 the processor further determining an amount of ammonia removed by the zirconium phosphate sorbent module.

4. The system of any of claims 1-3, wherein the processor estimates the patient pre-dialysis BUN level using an equation: $C_{Burea,\, pre} = \dfrac{(\bar{C}_{NH4} * V_{eff})\,/\,2}{V_{prp} - V_{post}(1 - URR)}$, wherein $C_{Burea,\, pre}$ is the patient pre-dialysis urea level, $\overline{C}_{NH4}$ is a volume averaged total ammonia content in the ammonium removal solution effluent; $V_{eff}$ is a volume of ammonium removal solutions introduced through the zirconium phosphate sorbent module, $V_{pre}$ is a patient water volume prior to a dialysis session, $V_{post}$ is a patient water volume after the dialysis session, and URR is a urea reduction ratio for the dialysis session.

5. The system of claim 4, wherein the processor is programmed to determine the urea reduction ratio using an equation: URR = 1 - e$^{-kt/v}$, wherein k is a dialyzer clearance, t is a length of time of a dialysis session, and v is a patient water volume.

6. The system of claim 1, wherein the processor is programmed to receive the total ammonia content of the ammonium removal solution effluent from the at least one ammonia sensor continuously during the method.

**EP 3 598 987 B1**

7. The system of claim 1, wherein the processor is programmed to receive the total ammonia content of the ammonium removal solution effluent from the at least one ammonia sensor at preset intervals.

8. The system of claim 2, wherein the processor controls the pump to first introduce water from the water source through the zirconium phosphate sorbent module, and then introduce brine from the brine source through the zirconium phosphate sorbent module.

9. The system of claim 8, wherein the processor is programmed to receive the total ammonia content of the ammonium removal solution effluent while water is introduced through the zirconium phosphate sorbent module and while brine is introduced through the zirconium phosphate sorbent module.

10. The system of claim 9, wherein the processor is programmed to receive the total ammonia content of the ammonium removal solution effluent while brine is introduced through the zirconium phosphate sorbent module.

11. The system of any of claims 1-10, further comprising a reservoir (107) fluidly connectable to the effluent line, the at least one ammonia sensor determining the total ammonia content of the ammonium removal solution effluent in the reservoir.

12. The system of any of claims 1-11, wherein the at least one ammonia sensor is in the effluent line.

13. The system of any of claims 1-12, further comprising a temperature sensor in communication with the processor.

14. A method, comprising:

introducing one or more ammonium removal solutions into a zirconium phosphate sorbent module (102);
determining a total ammonia content in an ammonium removal solution effluent of the zirconium phosphate sorbent module using an ammonia sensor (108); and
estimating a patient pre-dialysis BUN level based on the total ammonia content in the ammonium removal solution effluentpreferably wherein the ammonia sensor is in an effluent line
(109) fluidly connectable to an outlet (104) of the zirconium phosphate sorbent module preferably further comprising the step of introducing the ammonium removal solution effluent into a reservoir (107), and wherein the step of determining the total ammonia content in the ammonium removal solution effluent comprises determining the total ammonia content in the reservoirpreferably wherein the step of estimating the patient pre-dialysis BUN level comprises determining a total ammonia content of the ammonium removal solution effluent to determine a volume averaged total ammonia content of the ammonium removal solution effluent.

**Patentansprüche**

1. System, umfassend:
einen Ammoniumentfernungsströmungsweg (101), umfassend:

mindestens eine Ammoniumentfernungslösungsquelle (105);
wobei die Ammoniumentfernungslösungsquelle mit einem Einlass (103) eines Zirkoniumphosphatsorptionsmoduls (102) fluidverbindbar ist;
eine Pumpe (106); und
eine Abflussleitung (109), die mit einem Zirkoniumphosphatmodulauslass (104) fluidverbindbar ist;
mindestens einen Ammoniaksensor (108); und
einen Prozessor in Kommunikation mit dem mindestens einen Ammoniaksensor, wobei der Prozessor basierend auf einem Gesamtammoniakgehalt in einem Ammoniumentfernungslösungsabfluss des Zirkoniumphosphatsorptionsmittelmoduls einen BUN-Spiegel des Patienten vor der Dialyse schätzt.

2. System nach Anspruch 1, wobei das System ein Sorptionsmittelladegerät umfasst; und
wobei die mindestens eine Ammoniumentfernungslösungsquelle mindestens eine Wasserquelle (306) und eine Solequelle (305) umfasst.

3. System nach Anspruch 1 oder 2, wobei der Prozessor ferner eine durch das Zirkoniumphosphatsorptionsmittelmodul entfernte Ammoniakmenge bestimmt.

4. System nach einem der Ansprüche 1 bis 3, wobei der Prozessor den BUN-Spiegel des Patienten vor der Dialyse unter Verwendung einer Gleichung schätzt:

$$C_{CBHarnstoff,vor} = \frac{(\bar{C}_{NH4} * V_{eff})/2}{V_{prp} - V_{nach}(1-URR)},$$ wobei "$C_{BHarnstoff,\ vor}$" der Harnstoffspiegel des Patienten vor der Dialyse ist, $\bar{C}_{NH4}$ ein volumengemittelter Gesamtammoniakgehalt im Ammoniumentfernungslösungsabfluss ist; $V_{eff}$ ein durch das Zirkoniumphosphatsorptionsmittelmodul eingeführtes Volumen Ammoniumentfernungslösungen ist, $V_{vor}$ ein Wasservolumen des Patienten vor einer Dialyserunde ist, $V_{nach}$ ein Wasservolumen des Patienten nach der Dialyserunde ist und URR ein Harnstoffreduktionsverhältnis für die Dialyserunde ist.

5. System nach Anspruch 4, wobei der Prozessor dazu programmiert ist, das Harnstoffreduktionsverhältnis unter Verwendung einer Gleichung zu bestimmen:

URR = 1 - $e^{-kt/v}$, wobei k eine Dialysatorclearance ist, t eine Zeitdauer einer Dialyserunde ist und v ein Wasservolumen des Patienten ist.

6. System nach Anspruch 1, wobei der Prozessor dazu programmiert ist, während des Verfahrens von dem mindestens einen Ammoniaksensor kontinuierlich den Gesamtammoniakgehalt des Ammoniumentfernungslösungsabflusses zu empfangen.

7. System nach Anspruch 1, wobei der Prozessor dazu programmiert ist, von dem mindestens einen Ammoniaksensor den Gesamtammoniakgehalt des Ammoniumentfernungslösungsabflusses in voreingestellten Intervallen zu empfangen.

8. System nach Anspruch 2, wobei der Prozessor die Pumpe derart steuert, dass sie zuerst Wasser aus der Wasserquelle durch das Zirkoniumphosphatsorptionsmodul einbringt und dann Sole aus der Solequelle durch das Zirkoniumphosphatsorptionsmodul einbringt.

9. System nach Anspruch 8, wobei der Prozessor dazu programmiert ist, den Gesamtammoniakgehalt des Ammoniumentfernungslösungsabflusses zu empfangen, während Wasser durch das Zirkoniumphosphatsorptionsmodul eingebracht wird und während Sole durch das Zirkoniumphosphatsorptionsmodul eingebracht wird.

10. System nach Anspruch 9, wobei der Prozessor dazu programmiert ist, den Gesamtammoniakgehalt des Ammoniumentfernungslösungsabflusses zu empfangen, während Sole durch das Zirkoniumphosphatsorptionsmittelmodul eingebracht wird.

11. System nach einem der Ansprüche 1 bis 10, ferner umfassend ein Reservoir (107), das mit der Abflussleitung fluidverbindbar ist, wobei der mindestens eine Ammoniaksensor den Gesamtammoniakgehalt des Ammoniumentfernungslösungsabflusses in dem Reservoir bestimmt.

12. System nach einem der Ansprüche 1 bis 11, wobei sich der mindestens eine Ammoniaksensor in der Abflussleitung befindet.

13. System nach einem der Ansprüche 1 bis 12, ferner umfassend einen Temperatursensor in Kommunikation mit dem Prozessor.

14. Verfahren, umfassend:

Einbringen einer oder mehrerer Ammoniumentfernungslösungen in ein Zirkoniumphosphatsorptionsmittelmodul (102);
Bestimmen eines Gesamtammoniakgehalts in einem Ammoniumentfernungslösungsabfluss des Zirkoniumphosphatsorptionsmittelmoduls unter Verwendung eines Ammoniaksensors (108); und
Schätzen eines BUN-Spiegels eines Patienten vor der Dialyse basierend auf dem Gesamtammoniakgehalt im Ammoniumentfernungslösungsabfluss, wobei sich der Ammoniaksensor vorzugsweise in einer Abflussleitung (109) befindet, die mit einem Auslass (104) des Zirkoniumphosphatsorptionsmittelmoduls fluidverbindbar ist, vorzugsweise ferner umfassend den Schritt des Einbringens des Ammoniumentfernungslösungsabflusses in ein Reservoir (107), und wobei der Schritt des Bestimmens des Gesamtammoniakgehalts im Ammoniumentfernungslösungsabfluss das Bestimmen des Gesamtammoniakgehalts im Reservoir umfasst, wobei der Schritt des Schätzens des BUN-Spiegels des Patienten vor der Dialyse vorzugsweise das Bestimmen eines Gesamtammoniakgehalts des Ammoniumentfernungslösungsabflusses umfasst, um einen volumengemittelten Gesamtammoniakgehalt des Ammoniumentfernungslösungsabflusses zu bestimmen.

**Revendications**

1. Système comprenant :

   un chemin d'écoulement d'élimination d'ammonium (101) comprenant :

   au moins une source de solution d'élimination d'ammonium (105) ;
   la source de solution d'élimination d'ammonium pouvant être raccordée fluidiquement à une entrée (103) de module de sorbant de phosphate de zirconium (102) ;
   une pompe (106) ; et
   une conduite d'effluent (109) pouvant être raccordée fluidiquement à une sortie de module de phosphate de zirconium (104) ;

   au moins un capteur d'ammonium (108) ; et
   un processeur en communication avec l'au moins un capteur d'ammonium, le processeur estimant un niveau d'AUS pré-dialyse du patient sur la base d'une teneur totale en ammonium dans un effluent de solution d'élimination d'ammonium du module de sorbant de phosphate de zirconium.

2. Système selon la revendication 1, dans lequel le système comprend un chargeur de sorbant ; et
   dans lequel l'au moins une source de solution d'élimination d'ammonium comprend au moins une source d'eau (306) et une source de saumure (305).

3. Système selon les revendications 1 ou 2, le processeur déterminant en outre une quantité d'ammonium éliminée par le module de sorbant de phosphate de zirconium.

4. Système selon l'une quelconque des revendications 1 à 3, dans lequel le processeur estime le niveau d'AUS pré-dialyse du patient à l'aide d'une équation :

$$C_{\text{Burée,pré}} = \frac{(\bar{C}_{NH4} * V_{eff})/2}{V_{prp} - V_{post}(1 - TRU)},$$ où $C_{\text{Burée, pré}}$ est le niveau d'urée du patient pré-dialyse, $\overline{C}_{NH4}$ est une teneur totale moyenne en ammonium de l'effluent de solution d'élimination d'ammonium ;
   $V_{etf}$ est un volume de solutions d'élimination d'ammonium introduit à travers le module de sorbant de phosphate de zirconium, $V_{pré}$ est un volume d'eau du patient avant une séance de dialyse, $V_{post}$ est un volume d'eau du patient après la séance de dialyse, et TRU est un taux de réduction de l'urée pour la séance de dialyse.

5. Système selon la revendication 4, dans lequel le processeur est programmé pour déterminer le rapport de réduction de l'urée à l'aide d'une équation :
   TRU = 1 - $e^{-kt/v}$, où k est une clairance de dialyseur, t est la durée d'une séance de dialyse et v est un volume d'eau du patient.

6. Système selon la revendication 1, dans lequel le processeur est programmé pour recevoir la teneur totale en ammonium de l'effluent de solution d'élimination d'ammonium de l'au moins un capteur d'ammonium en continu pendant le procédé.

7. Système selon la revendication 1, dans lequel le processeur est programmé pour recevoir la teneur totale en ammonium de l'effluent de solution d'élimination d'ammonium de l'au moins un capteur d'ammonium à des intervalles prédéfinis.

8. Système selon la revendication 2, dans lequel le processeur commande la pompe pour introduire d'abord l'eau provenant de la source d'eau à travers le module de sorbant de phosphate de zirconium, puis pour introduire la saumure provenant de la source de saumure à travers le module de sorbant de phosphate de zirconium.

9. Système selon la revendication 8, dans lequel le processeur est programmé pour recevoir la teneur totale en ammonium de l'effluent de solution d'élimination d'ammonium tandis que de l'eau est introduite à travers le module de sorbant de phosphate de zirconium et tandis que de la saumure est introduite à travers le module de sorbant de phosphate de zirconium.

10. Système selon la revendication 9, dans lequel le processeur est programmé pour recevoir la teneur totale en ammonium de l'effluent de solution d'élimination d'ammonium tandis que de la saumure est introduite à travers le module de sorbant de phosphate de zirconium.

11. Système selon l'une quelconque des revendications 1 à 10, comprenant en outre un réservoir (107) pouvant être raccordé fluidiquement à la conduite d'effluent, l'au moins un capteur d'ammonium déterminant la teneur totale en ammonium de l'effluent de solution d'élimination d'ammonium dans le réservoir.

12. Système selon l'une quelconque des revendications 1 à 11, dans lequel l'au moins un capteur d'ammonium se trouve dans la conduite d'effluent.

13. Système selon l'une quelconque des revendications 1 à 12, comprenant en outre un capteur de température en communication avec le processeur.

14. Procédé comprenant :

l'introduction d'une ou plusieurs solutions d'élimination d'ammonium dans un module de sorbant de phosphate de zirconium (102) ;
la détermination d'une teneur totale en ammonium dans un effluent de solution d'élimination d'ammonium du module de sorbant de phosphate de zirconium à l'aide d'un capteur d'ammonium (108) ; et
l'estimation d'un niveau d'AUS pré-dialyse d'un patient sur la base de la teneur totale en ammonium de l'effluent de solution d'élimination d'ammonium, de préférence dans lequel le capteur d'ammonium se trouve dans une conduite d'effluent (109) pouvant être raccordée fluidiquement à une sortie (104) du module de sorbant de phosphate de zirconium comprenant en outre de préférence l'étape d'introduction de l'effluent de solution d'élimination d'ammonium dans un réservoir (107), et dans lequel l'étape de détermination de la teneur totale en ammonium de l'effluent de solution d'élimination d'ammonium comprend la détermination de la teneur totale en ammonium dans le réservoir, de préférence dans lequel l'étape d'estimation du niveau d'AUS pré-dialyse d'un patient comprend la détermination d'une teneur totale en ammonium de l'effluent de solution d'élimination d'ammonium pour déterminer une teneur totale moyenne en ammonium de l'effluent de solution d'élimination d'ammonium.

FIG. 1

Connect Zirconium Phosphate Sorbent Module to Ammonium Removal Flow Path — 201

Introduce Ammonium Removal Solution Through Zirconium Phosphate Sorbent Module — 202

Measure Total Ammonia Content of the Ammonium Removal Solution Effluent — 203

Estimate Patient Pre-Dialysis BUN Level — 204

FIG. 2

FIG. 3

```
┌─────────────────────────────────────────┐
│ Connect Zirconium Phosphate Sorbent Module │   401
│          to a Sorbent Recharger           │
└─────────────────────────────────────────┘
                     │
                     ▼
┌─────────────────────────────────────────┐
│     Introduce Water Through Zirconium     │   402
│         Phosphate Sorbent Module          │
└─────────────────────────────────────────┘
                     │
                     ▼
┌─────────────────────────────────────────┐
│      Introduce a Brine Solution Through    │   403
│    the Zirconium Phosphate Sorbent Module  │
└─────────────────────────────────────────┘
                     │
                     ▼
┌─────────────────────────────────────────┐
│      Rinse Zirconium Phosphate Sorbent     │   404
│           Module with Water               │
└─────────────────────────────────────────┘
                     │
                     ▼
┌─────────────────────────────────────────┐
│        Estimate the Patient Pre-dialysis   │   405
│                BUN Level                  │
└─────────────────────────────────────────┘
```

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62702497
- US 642847
- US 20150367055 A1